# EUROPEAN PATENT APPLICATION

(11) **EP 2 628 438 A1**
(43) Date of publication of application: **21.08.2013**
(21) Application number: 13154721.8
(22) Date of filing: 08.02.2013
(51) Int. Cl.: A61B 1/00, A61B 1/233, A61M 25/10

(54) **Expansion catheter**

(30) Priority: 16.02.2012 JP 2012031783
(71) Applicant: Terumo Kabushiki Kaisha, Tokyo 151-0072 (JP)
(72) Inventor: Kumoyama, Kenichi, Nakai-machi, Kanagawa 259-0151 (JP); Kinoshita, Yasushi, Fujinomiya-shi, Shizuoka 418-0015 (JP); Suehara, Satoru, Nakai-machi, Kanagawa 259-0151 (JP); Arastoo, Riyaheh, Nakai-machi, Kanagawa 259-0151 (JP)
(74) Representative: Dossmann, Gérard

(57) **Abstract**

Disclosed herein is an expansion catheter (100, 200) including: an expansion body (110, 210) which can be expanded; an elongate main body (120, 220) to a distal section to which the expansion body is attached, the main body comprising a bent section (125, 225); and an imaging section (160, 272) by which the expansion body at the distal end is imaged from a proximal side of the bent section of the main body. In the first embodiment the bent section is transparent and the imaging section is within the main body, in the second embodiment the image section (272) is attached externally to the main body.

## Description

The present disclosure contains subject matter related to that disclosed in Japanese Priority Patent Application JP 2012-031783 filed in the Japan Patent Office on February 16, 2012, the entire content of which is hereby incorporated by reference.

The present invention relates to an expansion catheter capable of expanding (dilating) a natural ostium stenosed due to sinusitis.

An inflammation generated in a nasal cavity due to common cold or allergy or the like may spread into a paranasal sinus, which is an intraosseous syrinx adjacent to the nasal cavity. Such an inflammation generated in the paranasal sinus is called sinusitis. The paranasal sinus is in communication with the nasal cavity through a small hole called natural ostium. When edema or thickening of a mucous membrane or the like is generated in the vicinity of the natural ostium due to sinusitis, the natural ostium is stenosed. As a result, it becomes difficult for secretion, bacteria and the like in the paranasal sinus to be discharged into the nasal cavity, and ventilation disorder is caused.

In the past, sinusitis has ordinarily been treated by a surgical procedure in which the stenosis in the natural ostium is removed by forceps, a drill or the like. In recent years, attention has been paid to a lowly invasive procedure in which the stenosed natural ostium is dilated by use of a balloon catheter. For instance, JP-T-2008-513125 discloses a procedure in which a natural ostium is dilated by endoscopically operating a balloon catheter.

When a balloon catheter is endoscopically operated, however, there arises the problem that the operator's hands are fully occupied or that the range of vision is limited by the diametral size of the endoscope. Taking these problems into consideration, the present inventors have been making research and development as to balloon catheters on which a miniaturized camera is mounted.

Where a camera is thus mounted on a balloon catheter, it is a common practice to provide the camera at the distal end of the catheter and thereby to check the forward side of the balloon, in order to look for the natural ostium. If the forward side of the balloon is only checked, however, it is very difficult to detect whether or not the balloon has been assuredly positioned in the stenosis part or whether or not the stenosis part has been successfully dilated by the balloon, though it is possible to check the arrival of the balloon catheter at the lesion part. It is considered that if the balloon can also be checked, it is possible to achieve the desired procedure accurately, safely and speedily.

It is an object of the present invention to provide an expansion catheter by which it is possible to check the forward side of an expansion body such as a balloon and also check the expansion body, thereby achieving an accurate, safe and speedy procedure.

According to an embodiment of the present invention, there is provided an expansion catheter including: an expansion body which can be expanded; an elongate main body to a distal section of which the expansion body is attached; and an imaging section by which the expansion body is imaged from a proximal side thereof.

The expansion catheter configured as above ensures that both the front side of the distal end of the main body and contour lines of the expansion body before and after expansion thereof are captured in the visual field of the imaging section. As a result, the operator is enabled to advance the expansion catheter to a target part in a patient's body while checking the condition on the forward side of the expansion catheter. It is also possible for the operator to expand (dilate) the target part while checking the position of the expansion body and the expanding operation. Therefore, such situations as deployment of the expansion body into a position different from an intended position and the attendant expansion of the expansion body in the erroneous position or a necessitated readjustment of the position of the expansion body can be obviated. Accordingly, the expansion catheter configured as above promises an accurate, safe and speedy procedure (treatment).

In the above-mentioned expansion catheter, a configuration may be adopted wherein the expansion catheter further includes an elongate body provided with the imaging section at a distal portion thereof; the main body is formed therein with a lumen in which the elongate body can be inserted and passed in a movable manner; and the imaging section images the expansion body from the proximal side when the distal portion of the elongate body is located at the bent section, and picks up an image of a forward side of the main body when the distal portion of the elongate body is located at the distal section of the main body. This configuration ensures that since the imaging section is provided on the elongate body movable within the main body, it is possible, by adjusting the position of the imaging section, to check the advancing direction of the expansion catheter and to check the contour lines of the expansion body before and after expansion thereof. Besides, since different parts can be checked by use of a single imaging section, the manufacturing cost of the expansion catheter can be reduced.

In the above-mentioned expansion catheter, a configuration may be adopted wherein the elongate body includes a first part corresponding to the distal section of the main body in a condition where the distal portion of the elongate body is located at the distal section of the main body, a second part corresponding to the bent section in the condition, and a third part located on the proximal side of the bent section in the condition; the first part has a plastically deformable structure or is formed of a plastically deformable material; and the second part has such a structure or is formed of such a material that is lower than the third part in rigidity. This configuration ensures that when the main body is advanced in a body lumen or the like in the condition where the distal portion of the elongate body is located at the distal section of the main body, the first part of the elongate body can easily be advanced in the body lumen or to a lesion part while deforming, since the first part is formed to be plastically deformable. Since the second part is lower than the third part in rigidity, the third part is not liable to be deformed and a pushing-in force can be easily transmitted therethrough, at the time when the distal portion of the elongate body is advanced within the main body to the distal section of the main body. On the other hand, the second part is deformed to be smoothly advanced, at the time of turning along the bent section.

In the above-mentioned expansion catheter, a configuration may be adopted wherein the expansion catheter further includes a distal imaging section which is attached to the distal section of the main body and which picks up an image of the distal side of the main body, and the imaging section is attached to the bent section. According to this configuration, the image on the advancing direction of the expansion catheter can assuredly be picked up by the distal imaging section provided at the distal section of the main body, whereas the image of the expansion body can be reliably picked up by the imaging section provided at the bent section.

Where the imaging section in the above-mentioned expansion catheter is sensitive to visible rays and the expansion body is colored, the colored expansion body can be clearly imaged.

Where the imaging section in the above-mentioned expansion catheter is sensitive to infrared rays, the expansion body can be clearly imaged by use of infrared rays.

In the above-mentioned expansion catheter, a configuration may be adopted wherein the distal section of the main body is lower in rigidity than the other section of the main body. This configuration ensures that the distal section is deformed and advancement is facilitated, at the time when the expansion catheter is advanced in a body lumen or to a lesion part, the distal section of the main body first.
FIG. 1 is a block diagram showing the general configuration of an expansion system according to a first embodiment of the present invention;
FIG. 2 is a sectional view showing the configuration of a balloon catheter in the first embodiment;
FIG. 3 is a view showing the manner in which the balloon catheter is advanced in a nasal cavity;
FIG. 4 is a view showing the manner in which the balloon catheter is positioned in a stenosed natural ostium;
FIG. 5 is a view showing the condition of the balloon catheter at the time of observing a balloon dilating the natural ostium;
FIGS. 6A to 6C are views illustrating examples of an image picked up by an imaging device, wherein FIG. 6A shows the condition where the balloon is inserted in the natural ostium, FIG. 6B shows the condition where the balloon is inflated, and FIG. 6C shows the condition where the balloon is deflated;
FIG. 7 is a view showing the manner in which the stenosed natural ostium is dilated by the balloon catheter;
FIG. 8 is a view showing the condition where the balloon catheter is removed from the nasal cavity;
FIG. 9 is a sectional view showing the configuration of a balloon catheter in a second embodiment;
FIG. 10 is a sectional view showing the configuration of a balloon catheter in a third embodiment;
FIG. 11 is a sectional view showing the configuration of a modification of the balloon catheter in the third embodiment; and
FIG. 12 is a view showing the configuration on the distal side of a balloon catheter in a fourth embodiment.

Now, some embodiments of the present invention will be described below referring to the drawings. For convenience of description, the dimensional ratios in the drawings may be exaggerated and, therefore, different from the actual ratios.

### <First Embodiment>

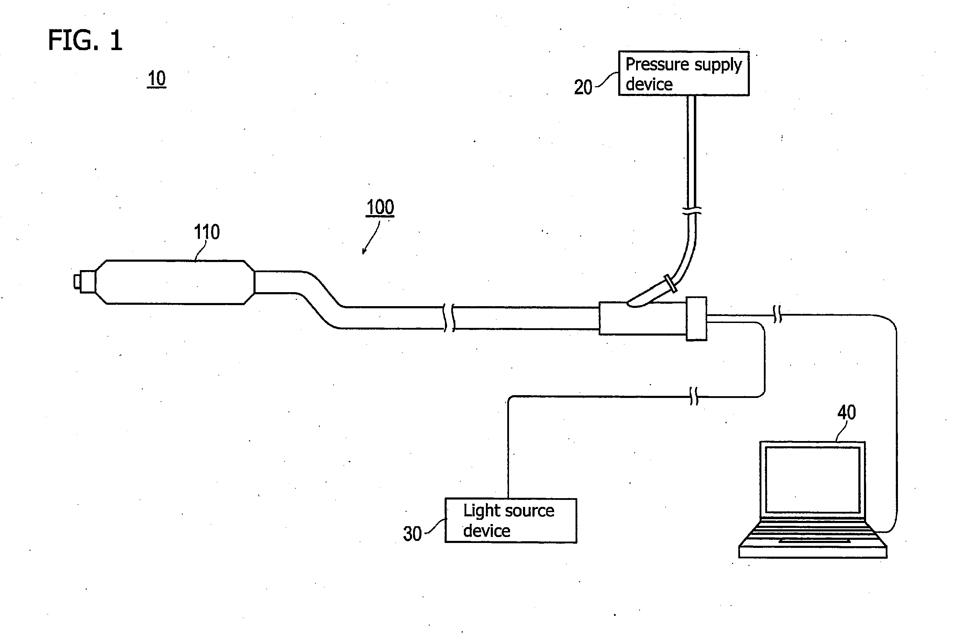

FIG. 1 is a block diagram showing the general configuration of an expansion system.

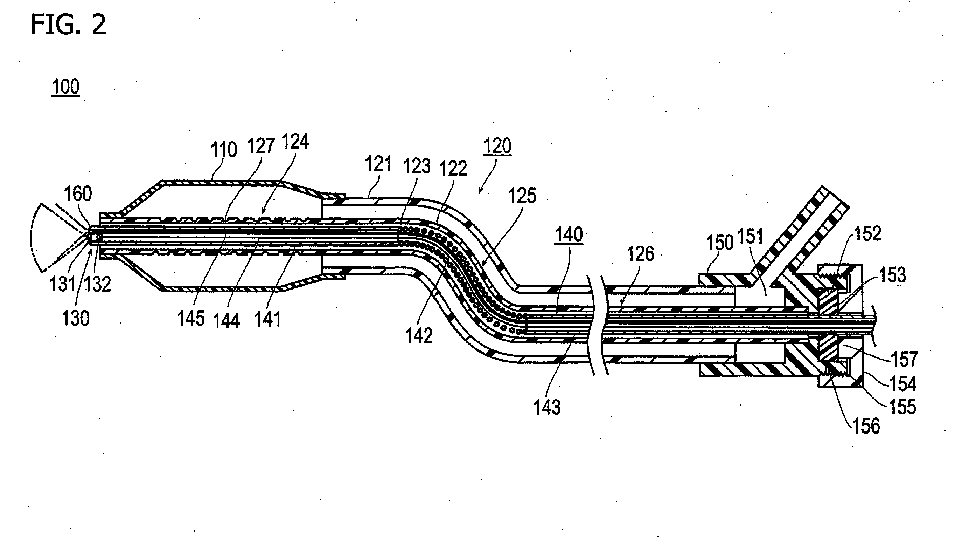

Outlining referring to FIGS. 1 and 2, the expansion system 10 of this embodiment for use in dilating a natural ostium stenosed due to sinusitis has a balloon catheter (expansion catheter) 100 which can be inserted into the natural ostium. In addition, the expansion system 10 in this embodiment has a pressure supply device 20, a light source device 30, and a display device 40 which are connected to the balloon catheter 100.

The pressure supply device 20 is in communication with the inside of a balloon (expansion body) 110 provided as part of the balloon catheter 100. The pressure supply device 20 supplies the balloon 110 with, for example, a liquid such as physiological saline and a contrast agent (radiopaque agent). The balloon 110 is inflated (expanded) when supplied with the pressurized liquid from the pressure supply device 20, and is deflated (contracted) when the liquid is discharged from the balloon 110 to the pressure supply device 20. The pressure supply device 20 is, for example, an indeflator, a syringe, a pump or the like.

The light source device 30 generates light which is irradiated from an illumination section 160 provided in the balloon catheter 100. The light generated in the light source device 30 is guided to the illumination section 160 by an optical fiber 145 disposed inside the balloon catheter 100.

The display device 40 displays an image picked up by an imaging section 130 provided in the balloon catheter 100. The imaging section 130 is composed of a lens 131 and an imaging element 132. The display device 40 is, for example, a PC (personal computer).

The structure of the balloon catheter 100 will be described more in detail.

FIG. 2 is a sectional view showing the configuration of the balloon catheter.

As shown in FIG. 2, the balloon catheter 100 includes the balloon 110 which can be inflated (expanded), and an elongate main body 120 to a distal portion of which the balloon 110 is attached. Besides, the balloon catheter 100 has a sonde catheter 140 which can be moved within the main body 120 and which is provided with the imaging section 130 at a distal portion thereof.

In this embodiment, the balloon 110 is colored, for easy checking thereof by the imaging section 130 as will be described later. The balloon 110 is formed from any of various resin materials which are generally used in the field of stent delivery systems. Specific examples of the material which can be adopted here include: polyamide resins including homopolymers such as polytetramethylene adipamide (nylon 46), polycaprolactam (nylon 6), polyhexamethylene adipamide (nylon 66), polyhexamethylene sebacamide (nylon 610), polyhexamethylene dodecamide (nylon 612), polyundecanolactam (nylon 11), polydodecanolactam (nylon 12), etc. and copolymers such as caprolactam/lauryllactam copolymer (nylon 6/12), caprolactam/aminoundecanoic acid copolymer (nylon 6/11), caprolactam/ω-aminononanoic acid copolymer (nylon 6/9), caprolactam/hexamethylenediammonium adipate copolymer (nylon 6/66), adipic acid-metaxylenediamine copolymer, copolymers of hexamethylenediamine and m- or p-phthalic acid, etc.; polyalkylene resins such as polyethylene resins such as linear low-density polyethylene (LLDPE), low-density polyethylene (LDPE), high-density polyethylene (HDPE), etc., polypropylene resins, etc.; polyolefins such as ethylene-propylene copolymer, ethylene-vinyl acetate copolymer, and their crosslinked products and partially cross-linked products (e.g., crosslinked type ethylene-vinyl acetate copolymer), etc.; epoxy resins; urethane resins; diallyl phthalate resins (allyl resin); polycarbonate resins; fluoro-resins; amino resins (urea resin, melamine resin, benzoguanamine resin); polyester resins (e.g., polyethylene terephthalate); styrol resins; acrylic resins; polyacetal resins; vinyl acetate resins; phenolic resins; vinyl chloride resins; silicone resins (silicon resins); polyarylene sulfides (e.g., polyphenylene sulfide); silicone rubbers; latex rubbers; and nylon elastomers, which are block copolymers having nylon 6, nylon 66, nylon 11, nylon 12 or the like as hard segment and having polyalkylene glycol, polyether, fatty polyester or the like as soft segment. Among these materials, preferred are those of high pressure resistance such as not to be burst when expanded (inflated), for example, polyamides, polyethylene terephthalate, high-density polyethylene and the like.

The main body 120 includes an outer tube 121 and an inner tube 122.

The outer tube 121 has the balloon 110 attached thereto so that its inside communicates with the inside of the balloon 110. The balloon 110 is attached to the outer circumference of a distal portion of the outer tube 121, namely, a distal section of the main body 120. The outer tube 121 is flexible, and, in this embodiment, it is light-transmitting. The outer tube 121 is preferably formed to be transparent, and formed, for example, from a thermoplastic resin such as polyolefins such as polyethylene, polypropylene, polybutene, ethylene-propylene copolymer, ethylene-vinyl acetate copolymer, ionomers, or mixtures of two or more of them, flexible polyvinyl chloride resins, polyamides, polyamide elastomers, polyesters, polyester elastomers, polyurethanes, fluoro-resins, acrylic resins, etc., silicone rubber, latex rubber or the like.

The inner tube 122 is disposed inside the outer tube 121. The inner tube 122 is formed therein with a lumen 123 in which the sonde catheter 140 can be inserted and passed in a movable manner. The inner tube 122 is flexible, and, in this embodiment, it transmits visible rays therethrough. The inner tube 122 is preferably formed to be transparent, and formed, for example, from any of the above-mentioned materials applicable to the outer tube 121. In addition, that portion of the inner tube 122 at which the balloon 110 is located on the radially outer side thereof (the distal section 124 of the main body 120) is formed with a spiral groove 127 by spiral processing, so as to be lower in rigidity than the other portion. This ensures that when that portion of the inner tube 122 which is lower in rigidity is deformed, the deformed shape will be maintained. The processing to which the above-mentioned portion of the inner tube 122 is subjected is not restricted to spiral processing; for example, the portion may be formed to have a bellows structure.

The main body 120, inclusive of the outer tube 121 and the inner tube 122, has a distal section 124 parallel to the extending direction of the balloon 110, and a bent section 125 which is located on the proximal side of the distal section 124 and which is bent to be non-parallel to the extending direction of the distal section 124. The main body 120 is, on the proximal side of the bent section 125, formed to extend substantially in parallel to the distal section 124. It is to be noted here, however, that since the main body 120 is flexible as a whole, its distal section 124 and its section 126 on the proximal side of the bent section 125 are not necessarily parallel to each other. Thus, in its fundamental posture with no load exerted thereon, the main body 120 is so formed that its sections on both sides of the bent section 125 are staggered from each other.

At the proximal end of the main body 120 is provided a hub 150, which is connected to the proximal ends of the outer tube 121 and the inner tube 122. The hub 150 is provided therein with a space (cavity) 151 which communicates with the outer tube 121. The space 151 is in communication with the pressure supply device 20. The liquid for inflating the balloon 110 is supplied from the pressure supply device 20 into the balloon 110 and is discharged from the balloon 110 to the pressure supply device 20 through the space 151 and the outer tube 121. The proximal end of the outer tube 121 is adhered to the hub 150 in a liquid-tight manner. Besides, the proximal end of the inner tube 122 protrudes from the proximal end of the outer tube 121, and is adhered to the hub 150 in a liquid-tight manner.

The hub 150 is formed with a recess 152 at the proximal end thereof, and an annular elastic body 153 is accommodated in the recess 152. The sonde catheter 140 can be inserted and passed in the annular elastic body 153. In addition, a holder 154 is attached to the proximal end of the hub 150 so that the elastic body 153 is accommodated in the recess 152. The holder 154 is provided with an inner screw 156 for screw engagement with an outer screw 155 formed at the outer circumference of the proximal end of the hub 150. Besides, the holder 154 is formed with a projected part 157 projected toward the elastic body 153. When an operator fastens the holder 154 to bring the hub 150 and the holder 154 closer to each other, the projected part 157 of the holder 154 presses the elastic body 153, thereby deforming the elastic body 153. When the elastic body 153 is thus deformed, its hole in which the sonde catheter 140 is inserted and passed is reduced in size, so that the position of the sonde catheter 140 is fixed by the deformed elastic body 153. When the holder 154 is unfastened, on the other hand, the deformation of the elastic body 153 is canceled, so that the sonde catheter 140 can be freely moved. The hub 150 and the holder 154 are each formed, for example, from a thermoplastic resin such as polycarbonates, polyamides, polysulfones, polyarylates, methacrylate-butylene-styrene copolymer, etc. In addition, the elastic body 153 is formed, for example, from silicone rubber, latex rubber or the like.

The sonde catheter 140 is inserted and passed in the inner tube 122 of the main body 120 via the hub 150. The sonde catheter 140 has a first part 141, a second part 142, and a third part 143 in this order from the distal end thereof, and the imaging section 130 is attached to the distal end of the first part 141.

The first part 141 is a part which is located inside the distal section 124 (the balloon 110) of the main body 120, in the condition where the distal portion of the sonde catheter 140 is located inside the distal section 124 of the main body 120. The first part 141 has a plastically deformable structure or is formed of a plastically deformable material. The first part 141 may have, for example, a meshed form obtained by weaving fibers in mesh form or providing a plurality of holes in the surface of a tubular member, or may have other form than the meshed form, such as a bellows form which is closed at an outer circumferential wall or a form having undergone spiral processing. The first part 141 is formed, for example, of a metallic material such as stainless steel or aluminum. When the first part 141 is deformed, the balloon 110 is also deformed following the first part 141. Plastic deformation of the first part 141 can be achieved not by the structure, such as the meshed structure, but by selection of material. For instance, the first part 141 may be formed of a metal which can easily be plastically deformed, such as aluminum.

The second part 142 is a part which is located inside the bent section 125 of the main body 120, in the condition where the distal portion of the sonde catheter 140 is located inside the distal section 124 of the main body 120. The second part 142 has elasticity such that its shape returns to an original shape when the position of the sonde catheter 140 is adjusted, even after the second part 142 is kept in a bent state relative to the first part 141 and the third part 143 while being inside the bent section 125. The second part 142 is formed, for example, as a metallic coil of stainless steel or the like.

The third part 143 is a part which is located on the proximal side of the bent section 125 of the main body 120, in the condition where the distal portion of the sonde catheter 140 is located inside the distal section 124 of the main body 120. In order that the third part 143 does not undergo plastic deformation under a force applied at the time of pushing in or pulling out the sonde catheter 140 in relation to the main body 120, the third part 143 has such a structure or is formed of such a material as to be higher than the second part 142 in rigidity. The third part 143 is formed, for example, as a metallic pipe of stainless steel or the like.

The imaging element 132 is sensitive to visible rays, and picks up an image. The imaging element 132 is, for example, a CCD image sensor or a CMOS image sensor. In this embodiment, the inner tube 122 and the outer tube 121 of the main body 120 are each formed of a visible ray-transmitting material. This ensures that the imaging section 130 can pick up an image of the outside of the main body 120 even when the imaging section 130 is located inside the main body 120, for example, inside the bent section 125.

In the sonde catheter 140, there are disposed a cable 144 for connection between the imaging element 132 and the display device 40, and optical fibers 145 for guiding light from the light source device 30 to the distal end of the sonde catheter 140. The cable 144 transmits the image picked up by the imaging section 130 to the display device 40. The plurality of optical fibers 145 are bundled by being accommodated in a tube disposed inside the sonde catheter 140, and irradiate light from their distal ends. The cable 144 and the optical fibers 145 are led out via the proximal end of the hub 150. A configuration may be adopted in which an optical fiber is used in place of the cable 144, and information captured at the distal end of the optical fiber is picked up as an image on the proximal side.

Now, an expanding (dilating) method in this embodiment conducted by use of the balloon catheter 100 will be described below.

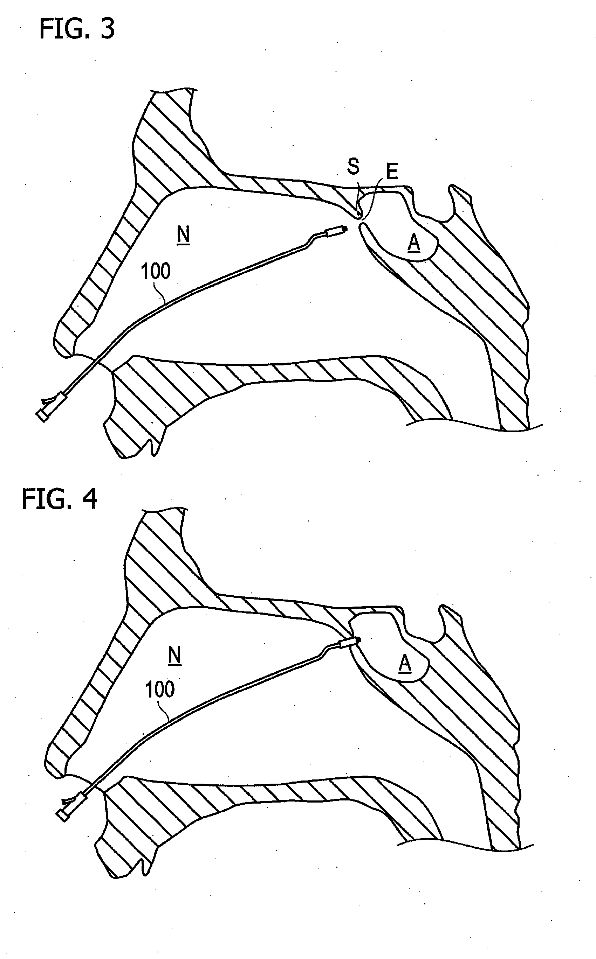

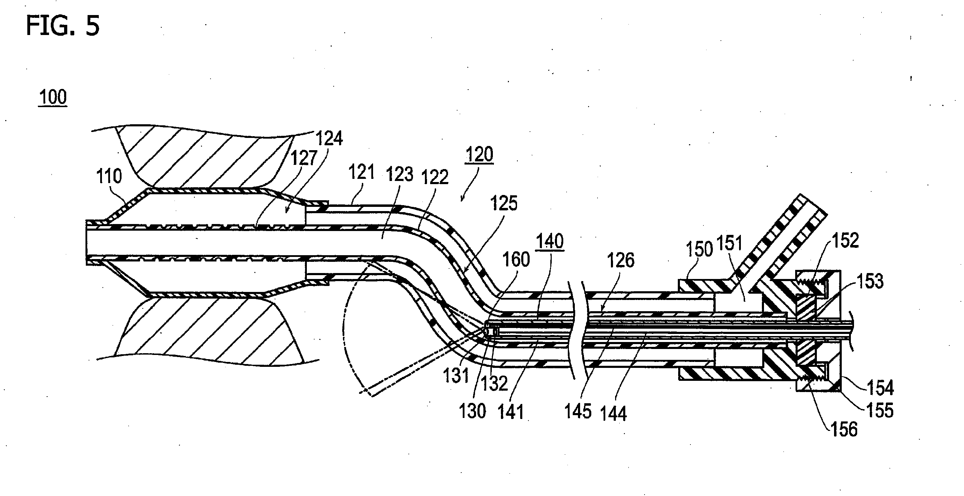

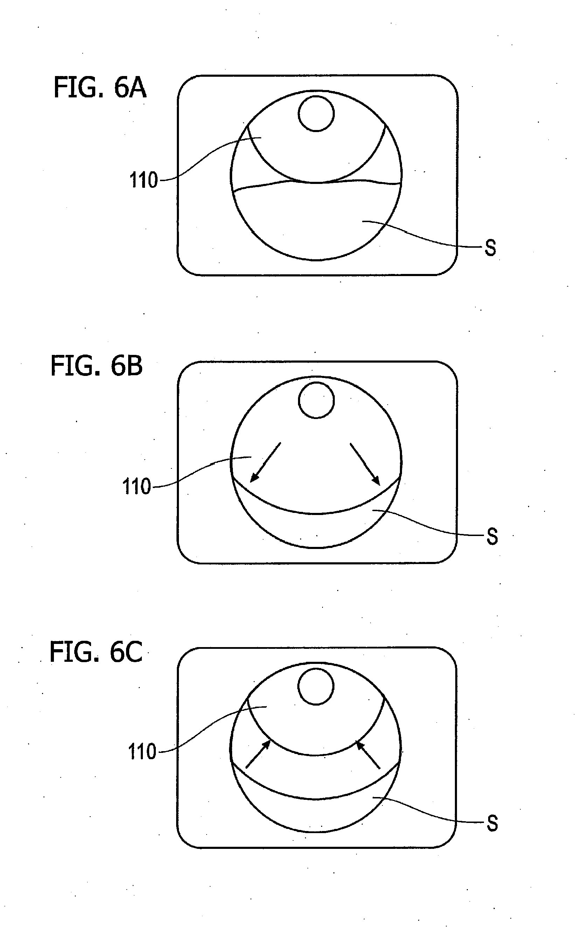

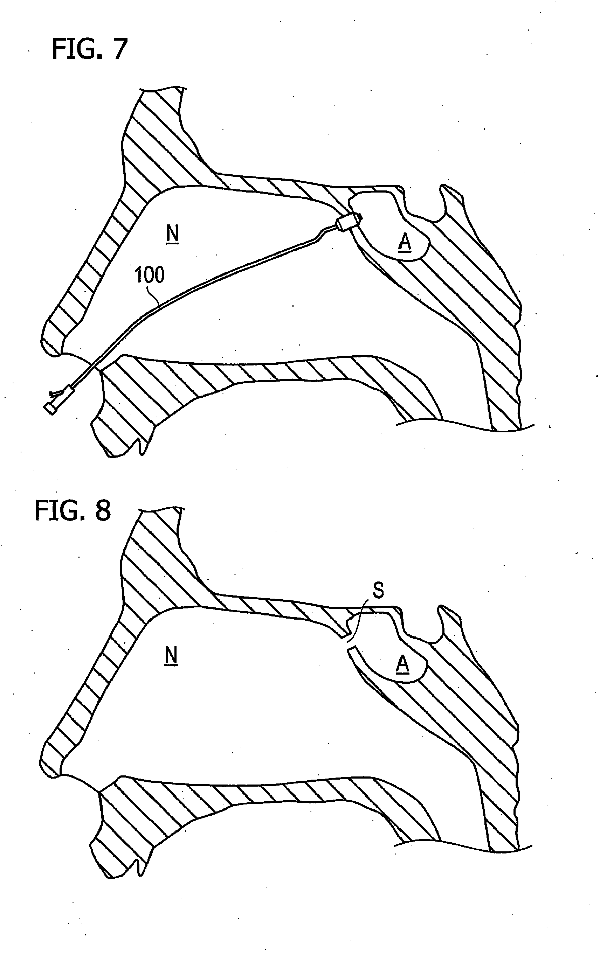

FIG. 3 is a view showing the manner in which the balloon catheter is advanced in a nasal cavity, FIG. 4 is a view showing the manner in which the balloon catheter is positioned to a stenosed natural ostium, FIG. 5 is a view showing the condition of the balloon catheter at the time of observing the balloon dilating the natural ostium, FIGS. 6A to 6C are views showing examples of the image picked up by the imaging element, FIG. 7 is a view showing the manner in which the stenosed natural ostium is dilated by the balloon catheter, and FIG. 8 is a view showing the condition where the balloon catheter has been removed from the nasal cavity.

Outlining the expanding method in this embodiment, the method includes: (1) a deployment step of deploying the balloon 110 into the vicinity of a target position in the patient's body while visually checking the forward side, in the condition where the distal portion of the sonde catheter 140 is located in the distal section 124 of the main body 120; (2) a visual checking step of visually checking the balloon 110 through the imaging section 130, by pulling the sonde catheter 140 proximally inside the main body 120 and thereby locating the imaging section 130 in the bent section 125, after the deployment step; (3) an adjustment step of adjusting the positions of the balloon 110 and the main body 120, based on the position of the balloon 110 detected by the visual checking step; and (4) an expansion step of expanding (inflating) the balloon 110, with the position of the main body 120 fixed. In addition, the expanding method in this embodiment further includes: (5) a contraction step of contracting (deflating) the balloon 110 while visually checking the contour line of the balloon 110, after the expansion step; and (6) a recovery step of recovering the balloon catheter 100, after the contraction step. Now, these steps will be described below, taking up as an example the expansion (dilation) of a natural ostium stenosed due to sinusitis.

As shown in FIGS. 3 and 4, in the deployment step, the operator advances the balloon catheter 100 inserted into the nasal cavity N toward the natural ostium E, and inserts the balloon 110 into the natural ostium E, thereby deploying (placing) the balloon 110 in the lesion part S. In the condition where the distal portion of the sonde catheter 140 is located in the distal section 124 of the main body 120, the holder 154 is fastened to the hub 150, whereby the position of the sonde catheter 140 is fixed. The balloon 110 is in a contracted (deflated) state.

In the condition where the light supplied from the light source device 30 through the optical fibers 145 is irradiated via the distal end of the sonde catheter 140, the operator advances the balloon catheter 100 toward the natural ostium E while checking the forward side of the balloon catheter 100 based on the image displayed on the display device 40. In addition, the operator inserts the balloon 110 into the natural ostium E and deploys the balloon 110 in the target site, while checking the front side of the balloon catheter 100 and the balloon 110 based on the image displayed on the display device 40.

As shown in FIG. 5, in the visual checking step, the operator unfastens the holder 154 from the hub 150, thereby making the sonde catheter 140 movable, and pulls the sonde catheter 140 proximally inside the main body 120, thereby locating the imaging section 130 inside the bent section 125. In the condition where the imaging section 130 is thus located in the bent section 125, the operator again fastens the holder 154 to the hub 150, thereby fixing the position of the sonde catheter 140. The imaging section 130 picks up the image of the balloon 110 and the natural ostium E from the proximal side, through the inner tube 122 and the outer tube 121 which transmit visible rays therethrough. The image picked up by the imaging section 130 is displayed on the display device 40, as an image which is like one of the images shown in FIGS. 6A to 6C.

In the adjustment step, the operator adjusts the position of the balloon 110 by moving the main body 120 while checking the image on the display device 40. The operator adjusts the position of the main body 120, for example, in such a manner that an image in which the balloon 110 and the natural ostium E make contact with each other, as shown in FIG. 6A, is obtained.

As shown in FIG. 7, in the expansion step, the operator expands (dilates) the natural ostium E by expanding (inflating) the balloon 110 through pressurizing the balloon 110 using the pressure supply device 20. When the balloon 110 is expanded (inflated), the natural ostium E is also expanded (dilated) following the external shape of the balloon 110, as indicated by arrows in FIG. 6B. The operator can adjusts the expansion (inflation) of the balloon 110 while checking the manner of expansion (dilation) of the natural ostium E, as shown in FIG. 6B, on the display device 40.

In the contraction step, the operator contracts the balloon 110 by decompressing the balloon 110 through the use of the pressure supply device 20. The operator confirms, on the display device 40, that only the external shape of the balloon 110 is contracted (deflated) as indicated by arrows in FIG. 6C, with the natural ostium E left expanded (dilated).

Thereafter, in the recovery step, as shown in FIG. 8, the operator removes the balloon 110 from the natural ostium E by pulling the balloon catheter 100, and the balloon catheter 100 is pulled out and recovered from the nasal cavity N.

The effects of this embodiment will be described.

In the balloon catheter 100 according to this embodiment, after the balloon 110 is positioned in a certain extent the sonde catheter 140 is pulled within the main body 120 to locate the imaging section 130 in the bent section 125. Since the bent section 125 extends to be non-parallel to the balloon 110, it faces the balloon 110, and the balloon 110 is imaged by the imaging section 130 from such a position as to face the balloon 110. This ensures that the balloon 110 can be securely imaged from a position where the balloon 110 is easily visible. As a result, the balloon 110 can be positioned in the natural ostium E accurately, safely and speedily, and the stenosed natural ostium E can be dilated (expanded) appropriately.

Especially, in this embodiment, the imaging section 130 is provided in the sonde catheter 140 which can be freely moved inside the main body 120. Therefore, the forward side of the balloon catheter 100 can be imaged when the imaging section 130 is located in the distal section 124 of the main body 120, and the state of the balloon 110 can be imaged from the proximal side thereof when the imaging section 130 is located in the bent section 125 of the main body 120. Since different parts can be checked by the single imaging section 130, the manufacturing cost of the expansion system 10 can be reduced.

In this embodiment, the sonde catheter 140 is divided into the first part 141, the second part 142, and the third part 143, which have different structures or are formed of different materials. When the main body 120 is advanced in a body lumen or the like in the condition where the distal portion of the sonde catheter 140 is located in the distal section 124 of the main body 120, the first part 141 can be easily advanced into the body lumen or to a lesion part while being deformed in shape, since the first part 141 is formed to be plastically deformable. In addition, since the second part 142 is lower in rigidity than the third part 143, it is ensured that when the distal portion of the sonde catheter 140 is advanced inside the main body 120 from the proximal side to the distal section 124 of the main body 120, the third part 143 is not liable to be deformed and a pushing-in force can be easily transmitted through the third part 143. On the other hand, the second part 142 can be smoothly advanced while deforming when it turns along the bent section 125.

The balloon 110 is colored, so that it is imaged by the imaging section 130 through the outer tube 121 and the inner tube 122 which are transparent. Therefore, the balloon 110 can be checked clearly.

In addition, the inner tube 122 of the main body 120 is so formed that its part (the distal section 124 of the main body 120) at which the balloon 110 is located on the outer circumference thereof is lower in rigidity than the other part. Therefore, when the first part 141 of the sonde catheter 140 is deformed in the condition where the distal portion of the sonde catheter 140 is located in the distal section 124 of the main body 120, the distal portion of the inner tube 122 is also deformed following the deformation of the first part 141. This ensures that the distal section of the main body 120 can be deformed into a desired shape permitting easy advancement into the diseased part, when carrying out the desired procedure. Accordingly, maneuverability of the balloon catheter 100 can be enhanced.

Incidentally, in the first embodiment as above, the inner tube 122 and the outer tube 121 are each formed of a transparent material through which visible rays can be transmitted. This, however, is not restrictive. For instance, the inner tube 122 and the outer tube 121 may each be formed of an opaque material. In this case, the inner tube 122 and the outer tube 121 are each provided with a transparent window part so that the balloon 110 can be observed from the imaging section 130 in FIG. 5. Or, alternatively, the inner tube 122 and the outer tube 121 may each be formed of a material which transmits infrared rays therethrough. In this case, the imaging element 132 is replaced by a camera sensitive to infrared rays, and light in a wavelength region including an infrared region is emitted from the light source device 30, whereby light of desired wavelength can be captured by the imaging element 132 and imaging can be achieved thereby.

### <Second Embodiment>

An expansion system according to a second embodiment differs from the expansion system of the first embodiment in a balloon catheter (expansion catheter) which can be inserted into a natural ostium E. The same configurations as those in the first embodiment will be described using the same reference symbols as used above.

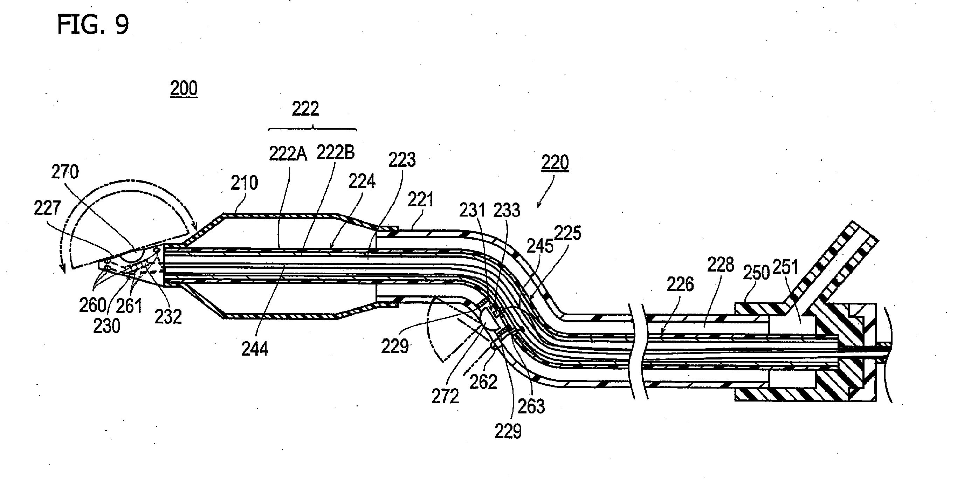

FIG. 9 is a sectional view showing the configuration of the balloon catheter in the second embodiment.

The expansion system in the second embodiment that can be used for expanding (dilating) a natural ostium E stenosed due to sinusitis has the balloon catheter (expansion catheter) 200 which can be inserted into the nasal ostium E. In addition, the expansion system in the second embodiment has a pressure supply device 20, a light source device 30, and a display device 40 which are connected to the balloon catheter 200.

The pressure supply device 20 is in communication with the inside of a balloon (expansion body) 210 provided as part of the balloon catheter 200. Incidentally, the pressure supply device 20 is the same as the pressure supply device 20 in the first embodiment above, and, therefore, description thereof is omitted here.

The light source device 30 generates light emitted from first illumination sections 260 and a second illumination section 262 which are provided as parts of the balloon catheter 200. The light generated by the light source device 30 is guided to the first illumination sections 260 and the second illumination section 262 by first optical fibers 261 and a second optical fiber 263 which are disposed inside the balloon catheter 200.

The display device 40 displays thereon images picked up by a first imaging section 230 and a second imaging section 231 which are provided at parts of the balloon catheter 200. The display device 40 can display thereon the images captured by a first imaging element 232 and a second imaging element 233. In addition, the display device 40 can also display thereon only a required part or parts of the images picked up by the first imaging element 232 and the second imaging element 233. The display device is, for example, a PC (personal computer).

The balloon catheter 200 includes an elongate main body 220, and a balloon 210 which is provided at the outer circumference of the main body 220 and which can be expanded (inflated). Besides, the balloon catheter 200 has a first lens 270 which captures, in its visual field, an image of the distal side of the balloon 210 provided on the main body 220 and the contour lines of the balloon 210 before and after expansion (inflation) thereof. The first imaging element 232 picks up an image through the first lens 270. The first illumination sections 260 emit light, thereby illuminating the subject to be imaged.

In this embodiment, the balloon 210 is colored so as to facilitate the checking by the first imaging section 230 and the second imaging section 231 which will be described later. The balloon 210 is the same in form as the balloon 110 in the first embodiment above, and, therefore, description thereof is omitted here.

The main body 220 includes a flexible outer tube 221, and a flexible inner tube 222 disposed inside the outer tube 221. In addition, the main body 220 includes a distal projected part 227 which is provided at the distal end of the inner tube 222 and which is projected axially to the distal side of the balloon 210, and a hub 250 connected to the proximal ends of the outer tube 221 and the inner tube 222.

The main body 220, inclusive of the outer tube 221 and the inner tube 222, includes a distal section 224 which is parallel to the extending direction of the balloon 210, and a bent section 225 which is located on the proximal side of the distal section 224 and which is bent to be non-parallel to the extending direction of the distal section 224. The main body 220 is formed to extend substantially in parallel to the distal section 224, on the proximal side of the bent section 225. It is to be noted here, however, that since the main body 220 is flexible as a whole, its distal section 224 and its section 226 on the proximal side of the bent section 225 are not necessarily parallel to each other. Thus, in its fundamental posture with no load exerted thereon, the main body 220 is so formed that its sections on both sides of the bent section 225 are staggered from each other.

To the outer tube 221 is attached a balloon 210 so that the inside of the outer tube 221 is in communication with the inside of the balloon 210. The balloon 210 is attached to the outer circumference of the distal end of the outer tube 221, namely, the distal section 224 of the main body 220. The outer tube 221 is formed, for example, from a thermoplastic resin such as polyolefins such as polyethylene, polypropylene, polybutene, ethylene-propylene copolymer, ethylene-vinyl acetate copolymer, ionomers, or mixtures of two or more of them, flexible polyvinyl chloride resins, polyamides, polyamide elastomers, polyesters, polyester elastomers, polyurethanes, fluoro-resins, acrylic resins, etc., silicone rubber, latex rubber or the like.

The inner tube 222 is disposed inside the outer tube 221. The inner tube 222 is formed therein with a lumen 223. The inner tube 222 is flexible.

The inner tube 222 is composed of an outer layer tube 222A and an inner layer tube 222B laid on each other. When the inner tube 222 is bent by operator's hands, the inner layer tube 222B is plastically deformed, thereby maintaining the bent shape. The inner layer tube 222B may have a meshed form obtained by weaving fibers in mesh form or providing a plurality of holes in the surface of a tubular member, or may have other form than the meshed form, such as a bellows form which is closed at an outer circumferential wall or a form provided with a spiral groove. The inner layer tube 222B is formed, for example, of a metallic material such as stainless steel. Plastic deformation of the inner layer tube 222B can be achieved not by the structure, such as the meshed structure, but by selection of material. For instance, the inner layer tube 222B may be formed of a metal which can easily be plastically deformed, such as aluminum. Or, alternatively, the plastic deformation of the inner layer tube 222B may be achieved by a method in which a bar-like metallic wire formed of the above-mentioned material is firmly attached to a part of the inner layer tube. As the material for forming the outer layer tube 222A, the same materials as those for the outer tube 221 are applicable. Thus, the inner layer tube 222B is plastically deformed and can maintain the deformed shape, and the outer layer tube 222A is deformed following the deformation of the inner layer tube 222B. This permits the operator to adjust the shape of the inner tube 222 in conformity with the shape of the natural ostium E, or the like.

In the inside of the inner tube 222, there extend a first cable 244 and a second cable 245 through which the first imaging section 230 and the second imaging section 231 are connected to the display device 40, and the first optical fiber 261 and the second optical fiber 263 by which light is guided from the light source device 30 to the first illumination sections 260 and the second illumination section 262. The first optical fiber 261 and the second optical fiber 263 are led out from the proximal end of a hub 250.

The hub 250 is provided therein with a space (cavity) 251 communicating with a lumen 228 of the outer tube 221. The space 251 is in communication with the pressure supply device 20. A liquid for expanding (inflating) the balloon 210 is supplied from the pressure supply device 20 into the balloon 210 and is discharged from the balloon 210 to the pressure supply device 20, through the space 251 and the lumen 228. The proximal end of the outer tube 221 is adhered to the hub 250 in a liquid-tight manner, and the proximal end of the inner tube 222 is protruded from the proximal end of the outer tube 221 and adhered to the hub 250 in a liquid-tight manner.

The distal projected part 227 is formed in a tapered shape which broadens along the direction from the distal end toward the balloon 210. The distal projected part 227 is fixed to the distal end of the inner tube 222, and holds the first lens 270, the first illumination sections 260, and the first imaging element 232.

The hub 250 and the distal projected part 227 are each formed, for example, of a thermoplastic resin such as polycarbonates, polyamides, polysulfones, polyarylates, and methacrylate-butylene-styrene copolymer.

The first lens 270 is provided along the inclination of the tapered shape of the distal projected part 227. The first lens 270 captures in its visual field an image of the forward side of the distal end of the distal projected part 227 and, preferably, also captures the distal end of the distal projected part 227. In addition, the first lens 270 captures in its visual field the contour line of the balloon 210 before expansion (inflation) and, preferably, captures the contour lines of the balloon 210 before and after expansion (inflation). The contour line of the balloon 210 captured in the visual field of the first lens 270 is preferably an arcuate contour line constituting the outer circumference of the largest-diameter portion of the tapered part increasing in diameter along the direction from the distal end toward the proximal side, or an arcuate contour line constituting the outer circumference of a roughly cylindrical portion formed on the proximal side of the tapered part. The first lens 270 is preferably a fish-eye lens having a visual field of not less than 180 degrees, or other wide angle lens having a visual field of nearly 180 degrees.

The first illumination sections 260 are provided in plurality so that light can be irradiated in a wide range according to the first lens 270 which has the wide visual field.

The above-mentioned lens in this embodiment is not restrictive. It suffices for the first lens 270 to be capable of capturing in its visual field the forward side of the distal end of the distal projected part 227 and, preferably, also capturing the distal end of the distal projected part 227. In addition, the first lens 270 in this embodiment is not restricted to the fish-eye lens having a visual field of not less than 180 degrees or the other wide angle lens having a visual field of nearly 180 degrees, but may be a standard lens narrower in visual field than the fish-eye lens or wide angle lens.

The first imaging element 232 is, for example, a CCD image sensor or a CMOS image sensor. The first imaging element 232 may be composed of a single sensor, or may be composed of a plurality of sensors arrayed with each other correspondingly to the first lens 270 having the wide visual field.

The second lens 272 is provided on the outer tube 221. The second lens 272 is provided at a position, facing the balloon 210, of the bent section 225 so that the balloon 210 is captured in its visual field. It suffices for the second lens 272 to be able to capture the balloon 210 in its visual field. Thus, the second lens 272 may be, for example, a standard lens narrower in visual field than fish-eye lenses or wide angle lenses as above-mentioned. The second lens 272 captures in its visual field the contour line of the balloon 210 before expansion (inflation), preferably, the contour lines of the balloon 210 before and after expansion (inflation).

In addition, the second lens 272 is not restricted to the standard lens, but may be a fish-eye lens or wide angle lens as above-mentioned. In this case, the visual field is wider than that of a standard lens, so that the balloon 210 and the vicinity thereof can be checked in a wider range, and the proximal side of the second lens 272 can also be checked.

The second illumination section 262 is provided on the outer tube 221. The second illumination section 262 illuminates the contour line of the balloon 210 and the vicinity thereof, which are captured in the visual field of the second lens 272. For instance, when the balloon 210 is disposed in a stenosed natural ostium E, the second illumination section 262 illuminates the stenosed natural ostium E in contact with the balloon 210, in addition to the contour line of the balloon 210. The second illumination section 262 is preferably provided on the proximal side of the second lens 272. Where the second illumination section 262 is provided on the proximal side of the second lens 272, the second illumination section 262 does not obstruct the visual field of the second lens 272, so that the contour line of the balloon 210 can be visually checked more easily.

The second optical fiber 263 for guiding light to the second illumination section 262 penetrates the outer circumference of the inner tube 222, to be led into the space inside the outer tube 221. In order that the liquid moved inside the outer tube 221 for inflation and deflation of the balloon 210 would not flow into the inside of the inner tube 222, the through-hole formed in the outer circumference of the inner tube 222 so as to lead out the second optical fiber 263 is sealed in a liquid-tight manner. The second optical fiber 263 is led out from the proximal end of the hub 250, and optically connects the second illumination section 262 and the light source device 30 to each other.

The second imaging section 231 is disposed on the inner tube 222. The second imaging element 233 picks up an image in the visual field of the second lens 272, from the proximal side of the balloon 210. The second imaging element 233 is, for example, a CCD image sensor or a CMOS image sensor. The second imaging element 233 is disposed inside the outer tube 221 in the state of being surrounded by partition walls 229 so that the liquid for inflation and deflation of the balloon 210 would not flow thereinto.

The second cable 245 for transmitting a signal from the second imaging element 233 to the display device 40 penetrates the outer circumference of the inner tube 222, to be electrically connected to the second imaging element 233. The through-hole formed in the outer circumference of the inner tube 222 so as to pass the second cable 245 therethrough is sealed in a liquid-tight manner so that the liquid moved inside the outer tube 221 would not flow into the inner tube 222. The second cable 245 is led out from the proximal end of the hub 250, and electrically connects the second imaging element 233 and the display device 40 to each other. A configuration may be adopted in which an optical fiber is used in place of the second cable 245, and information captured at the distal end of the optical fiber is picked up as an image on the proximal side.

The effects of this embodiment will be described.

The balloon catheter 200 according to the second embodiment has the second imaging section 231 for imaging the balloon 210 from a position, facing the balloon 210, of the bent section 225. Therefore, an image of the balloon 210 can be securely picked up on the proximal side. This permits the operator to expand (dilate) the stenosed natural ostium E while checking the position of the balloon 210 and the expanding operation through the second imaging section 231, after advancing the balloon catheter 200 to the desired natural ostium E (see FIG. 4). Therefore, for example, deployment of the balloon 210 into a position different from the intended position as well as the resultant expansion (inflation) of the balloon 210 in the erroneous position or a readjustment of the position of the balloon 210 can be avoided. Consequently, the desired procedure can be achieved accurately, safely and speedily.

In addition, the first imaging section 230 different from the second imaging section 231 provided at the bent section 225 is provided on the distal side of the main body 220. Therefore, the forward side of the distal projected part 227 can be observed through the first imaging section 230, without need to move the imaging section as in the first embodiment. Besides, the balloon 210 can be observed from the proximal side through the second imaging section 231, whereby operability is enhanced. Incidentally, the forward side of the distal projected part 227 may be observed by use of the second imaging section 231, instead of observing the forward side of the distal projected part 227 by use of the first imaging section 230. Accordingly, a configuration may be adopted in which the first imaging section 230 is not provided and only the second imaging section 231 is provided.

Furthermore, the use of the first imaging section 230 enables not only observation of the forward side of the distal projected part 227 but also observation of the balloon 210 from the distal side. Therefore, it is possible to observe the distal side of the balloon 210 through the first imaging section 230 while observing the proximal side of the balloon 210 through the second imaging section 231. Accordingly, the position of the balloon 210 and the expanding operation can be checked more assuredly.

In addition, the imaging section is not restricted to the configuration in which an imaging element such as a CCD image sensor is used as in the first and second embodiments. For example, a bundle of optical fibers capable of receiving light emitted through the lens may be used as the imaging section.

### <Third Embodiment>

An expansion system according to a third embodiment differs from those in the first embodiment and the second embodiment in a balloon catheter (expansion catheter) which can be inserted into a natural ostium E. Especially, a configuration on the distal side of the balloon catheter 300 is different from those in the first embodiment and the second embodiment. Therefore, the configuration on the distal side of the balloon catheter 300 will be described particularly.

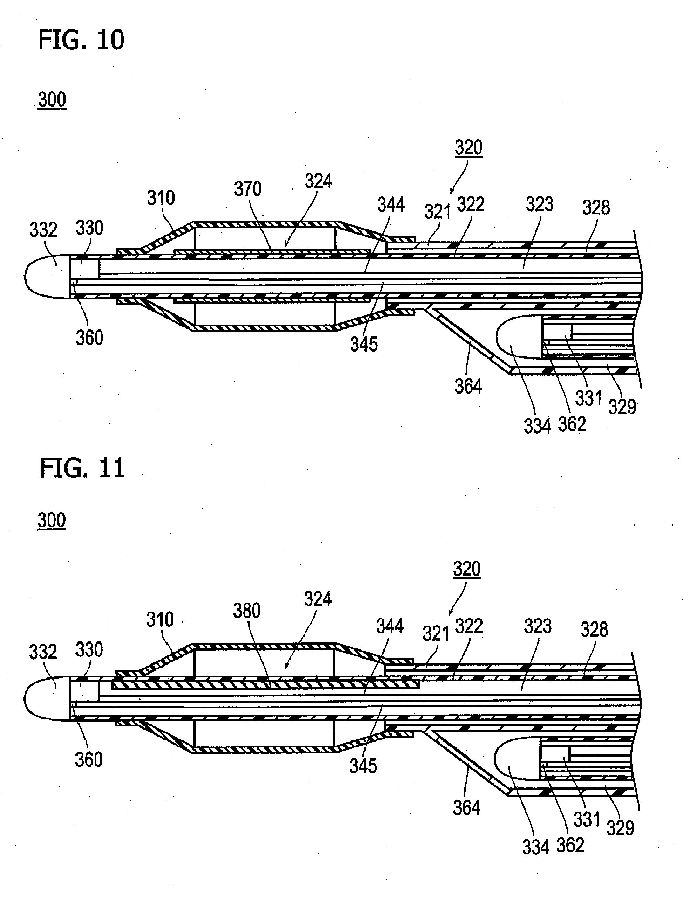

FIG. 10 is a view showing the configuration on the distal side of the balloon catheter.

Like in the first embodiment and the second embodiment, the balloon catheter 300 includes a balloon 310, and an elongate main body 320 having the balloon 310 attached to a distal section 324 thereof. In FIG. 10, the configuration on the proximal side is omitted from the illustration here. On the proximal side, a hub is provided and the elongate main body 320 is held by the hub, like in the first embodiment and the second embodiment. The elongate main body 320 includes an outer tube 321, and an inner tube 322 disposed inside the outer tube 321.

The inner tube 322 is disposed in a lumen 328 of the outer tube 321. In a lumen 323 of the inner tube 322, a cable 344 and an optical fiber 345 which extend from the proximal end are passed. At the distal end of the cable 344 is provided a first imaging section 330. The first imaging section 330 can image the condition on the distal side of the balloon catheter 300. At the distal end of the cable 344 is also provided an illumination section 360. The illumination section 360 irradiates light to the distal side of the balloon catheter 300, thereby to assist the imaging by the first imaging section 330.

In addition, at the distal ends of the first imaging section 330 and the illumination section 360 is provided a transparent distal chip 332. The distal chip 332 is rounded off at its distal end so that the distal end of the balloon catheter 300 is smoothly inserted into a body lumen.

The outer tube 321 is provided therein with a lumen 329, separately from the lumen 328. In the lumen 329 is disposed a second imaging section 331. The second imaging section 331 is provided on the proximal side of a distal section 324 (the balloon 310) of the main body 320 and on the outer circumference side (radially outer side) in relation to the distal section 324. The second imaging section 331 can image the condition of the balloon 310, through an observation window 364 which is provided as part of the outer tube 321 and is formed of a resin that is transparent to such an extent as to permit visual checking of the forward side therethrough. In the lumen 329 is provided an illumination section 362. The illumination section 362 irradiates light, which is transmitted through an optical fiber extending from the proximal end, toward the balloon 310 through the observation window 364. A distal chip 334 may be provided also at the distal ends of the second imaging section 331 and the illumination section 362. The distal end of the lumen 329 is sealed in a liquid-tight manner.

In the first embodiment, the inner tube 122 has been formed with the spiral groove 127, at the distal section 124 of the main body 120. In the third embodiment, a cover 370 composed of metallic mesh is provided on the outer circumference of the inner tube 322, instead of forming the inner tube 322 with a spiral groove. This ensures that when the portion of interest of the inner tube 322 which is lower in rigidity is deformed, the shape after the deformation is maintained.

The effects of this embodiment will now be described. It is to be noted that the same effects as those of the first embodiment and the second embodiment are omitted from the description here.

In the third embodiment, the second imaging section 331 is provided on the proximal side and on the outer circumference side (radially outer side) in relation to the distal section 324 of the main body 320. Therefore, a contracted (deflated) state and an expanded (inflated) state of the balloon 310 can be observed assuredly. While the main body has been bent in the first embodiment, a configuration wherein the outer tube 322 is provided therein with another lumen 329 and the second imaging section 331 is accommodated in the lumen 329 is adopted in the third embodiment. This ensures, for example, that a tube member having the lumen 328 and a tube member having the lumen 329 are joined in parallel to each other, whereby the outer tube 322 can be formed. Therefore, the outer tube 322 can be easily produced without bending any tube member.

In addition, in the third embodiment, the cover 370 composed of metallic mesh is attached to the outer circumference of the inner tube 322. This ensures that when the inner tube 322 low in rigidity is deformed at the distal section 324 of the main body 320, the deformation is maintained by the cover 370. Therefore, when the distal section 324 of the main body 320 is deformed, for example, in the case of advancing the balloon catheter 300 in a body lumen, the deformation is maintained. Consequently, advancement of the balloon catheter 300 is facilitated.

Besides, in the third embodiment, the distal end of the lumen 329 is sealed in a liquid-tight manner. This prevents internal matter, such as mucous membrane or nasal discharge, from being deposited on the second imaging section 331. Therefore, the second imaging section 331 can be used repeatedly, if necessary.

In the third embodiment shown in FIG. 10, the cover 370 is mounted onto the inner tube 322. In place of this configuration, another configuration can also be adopted to maintain the deformation of the distal section 324 of the main body 320.

FIG. 11 is a view showing a modification of the balloon catheter of the third embodiment.

In the balloon catheter 300 shown in FIG. 11, a metallic wire 380 is mounted in the inner tube 322, in place of the cover 370 shown in FIG. 10. The metallic wire 380 is higher than the inner tube 322 in rigidity. When the distal section 324 of the main body 320 is deformed, the deformation is maintained by the metallic wire 380.

### <Fourth Embodiment>

In an expansion system according to a fourth embodiment, the balloon catheter (expansion catheter) in the third embodiment is partly improved. Particularly, the expansion system in the fourth embodiment differs from those in the first embodiment and the second embodiment in the configuration on the distal side of the balloon catheter 300. Therefore, the configuration on the distal side of the balloon catheter 300 will be described especially.

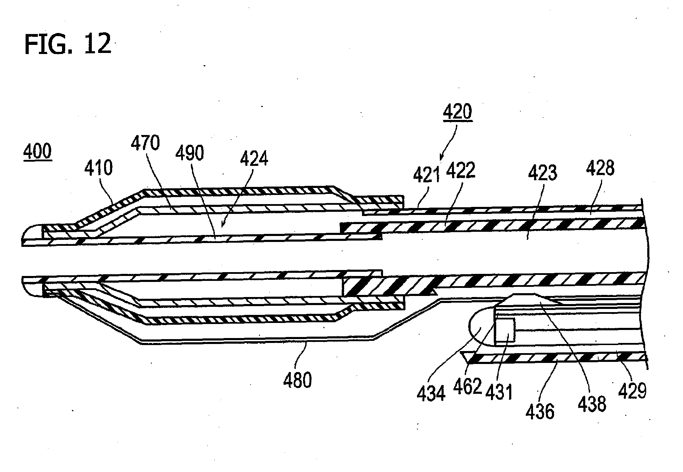

FIG. 12 is a view showing the configuration on the distal side of the balloon catheter in the fourth embodiment.

Like in the first embodiment and the second embodiment, a balloon catheter 400 includes a balloon 410, and an elongate main body 420 having the balloon 410 attached to a distal section 424 thereof. In FIG. 12, the configuration on the proximal side is omitted from the illustration. On the proximal side, a hub is provided and the elongate main body 420 is held by the hub, like in the first embodiment and the second embodiment. The elongate main body 420 includes an outer tube 421, and an inner tube 422 disposed inside the outer tube 421. A lumen 428 of the outer tube 421 is connected to the balloon 410, which is inflated when the lumen 428 is filled with air.

The inner tube 422 is disposed in the lumen 428 of the outer tube 421. None of a first imaging section, a cable and an illumination section is disposed in a lumen 423 of the inner tube 422, unlike in the third embodiment. The inner tube 422 is opened on the distal side; on the proximal side, on the other hand, the inner tube 422 is connected to a suction pump (not shown) which generates a suction force. The proximal end of the inner tube 422 extends proximally beyond the hub, and can be connected to the suction pump. With the suction pump operated, discretion or a body fluid or the like can be sucked in via the distal end of the inner tube 422.

The outer tube 421 is provided therein with a lumen 429, separately from the lumen 428. In the lumen 429 is disposed a second imaging section 431. The second imaging section 431 is provided on the proximal side of a distal section 424 (the balloon 410) of the main body 420 and on the outer circumference side (radially outer side) in relation to the distal section 424. The second imaging section 431 is attached to the distal end of a cable extending from the proximal end, and transmits a picked-up image to the proximal side through the cable.

Besides, in the lumen 429 is provided an illumination section 462. The illumination section 462 irradiates light, which is transmitted through an optical fiber extending from the proximal side, toward the balloon 410. A distal chip 434 may be provided at the distal ends of the second imaging section 431 and the illumination section 462. The lumen 429 is opened at the distal end thereof.

The second imaging section 431, the distal chip 434 and the illumination section 462 may be formed integrally by providing a casing 436. In this case, as shown in FIG. 12, a guide 438 is provided at the outer circumference of the casing 436. The guide 438 is provided therein with a through-hole, through which a wire 480 is passed. The guide 438 can be moved toward the distal side or the proximal side along the wire 480. In other words, the second imaging section 431 can be moved distally or proximally according to the movement of the guide 438. The wire 480 is made of a metal, for example.

Besides, in the fourth embodiment, the inner tube 422 does not extend to the distal end of the main body 424, but is opened in the vicinity of a proximal portion of the balloon 410. An auxiliary tube 490 is attached to the distal end of the inner tube 422. The auxiliary tube 490 is formed of a material which is higher in flexibility than the materials of the outer tube 421 and the inner tube 422. A cover 470 composed of metallic mesh is provided around the outer circumference of the auxiliary tube 490. While the auxiliary tube 490 is deformed easily, the cover 470 can hold the shape after the deformation.

The effects of this embodiment will now be described. It is to be noted that the same effects as those of the first to third embodiments are omitted from the description here.

In the fourth embodiment, the inner tube 422 is opened at the distal end thereof. This ensures that the lumen 423 of the inner tube 422 of the balloon catheter can be used for suction. With suction through the inner tube 422, secretion or the like in the living body can be removed, so that advancement of the balloon catheter in the living body can be facilitated. Besides, since no first imaging section is present, the distal end of the balloon catheter can be made smaller in diameter accordingly.

In addition, the second imaging section 431 can move the lumen 429 forward and rearward along the wire 480 by the guide 438. With the position of the second imaging section 431 changed selectively, it is possible to observe the balloon 410 and the living body through the second imaging section from an optimal site.

While nothing is disposed inside the inner tube 422 in the fourth embodiment, electrodes for treating a lesion part or the like may, for example, be supplied to the lesion part through the inner tube 422.

Incidentally, the expansion catheter according to the present invention is not restricted to the above-described embodiments, and, naturally, various modifications and alterations are possible within the scope of the present invention.

## Claims

1. An expansion catheter comprising:
an expansion body which can be expanded;
an elongate main body to a distal section of which the expansion body is attached; and
an imaging section by which the expansion body is imaged from a proximal side thereof.

2. The expansion catheter according to claim 1,
wherein the main body includes the distal section to an outer circumference of which the expansion body is attached and which extends along the expansion body, and a bent section which is located on the proximal side of the distal section and which is bent to be non-parallel to an extending direction of the distal section; and
the imaging section images the expansion body from a position, facing the expansion body, of the bent section.

3. The expansion catheter according to claim 2,
wherein the expansion catheter further comprises an elongate body provided with the imaging section at a distal portion thereof;
the main body is formed therein with a lumen in which the elongate body can be inserted and passed in a movable manner; and
the imaging section images the expansion body from the proximal side when the distal portion of the elongate body is located at the bent section, and picks up an image of a forward side of the main body when the distal portion of the elongate body is located at the distal section of the main body.

4. The expansion catheter according to claim 3,
wherein the elongate body includes a first part corresponding to the distal section of the main body in a condition where the imaging section is located at the distal section of the main body, a second part corresponding to the bent section in the condition, and a third part located on the proximal side of the bent section in the condition;
the first part has a plastically deformable structure; and
the second part has such a structure that the second part is lower than the third part in rigidity.

5. The expansion catheter according to claim 2,
wherein the elongate body includes a first part corresponding to the distal section of the main body in a condition where the imaging section is located at the distal section of the main body, a second part corresponding to the bent section in the condition, and a third part located on the proximal side of the bent section in the condition;
the first part is formed of a plastically deformable material; and
the second part is formed of such a material that the second part is lower than the third part in rigidity.

6. The expansion catheter according to claim 2,
wherein the expansion catheter further comprises a distal imaging section which is attached to the distal section of the main body and which picks up an image of the distal side of the main body, and
the imaging section is attached to the bent section.

7. The expansion catheter according to claim 2,
wherein the expansion catheter further comprises a distal imaging section which is attached to the distal section of the main body and which picks up an image of the distal side of the main body, and
the imaging section is provided on the proximal side and on an outer circumference side in relation to the distal section of the main body.

8. The expansion catheter according to any one of claims 1 to 7,
wherein the imaging section is sensitive to visible rays, and
the expansion body is colored.

9. The expansion catheter according to any one of claims 1 to 7,
wherein the imaging section is sensitive to infrared rays.

10. The expansion catheter according to any one of claims 1 to 9,
wherein the distal section of the main body is lower in rigidity than the other section of the main body.

11. The expansion catheter according to any one of claims 1 to 10,
wherein the main body is provided therein with a lumen for suction.
